# EUROPEAN PATENT SPECIFICATION

(11) **EP 3 437 672 B1**
(45) Date of publication and mention of the grant of the patent: **03.03.2021**
(21) Application number: 17775595.6
(22) Date of filing: 31.03.2017
(51) Int. Cl.: A61M 1/36, A61M 1/18, A61M 1/34

(54) **BLOOD PURIFICATION SYSTEM AND METHOD FOR PRIMING OF SAME**
BLUTREINIGUNGSSYSTEM UND -VERFAHREN ZUM PRIMING DAVON
SYSTÈME DE PURIFICATION DU SANG ET PROCÉDÉ D'AMORÇAGE ASSOCIÉ

(30) Priority: 31.03.2016 JP 2016073642; 31.03.2016 JP 2016073635
(43) Date of publication of application: 06.02.2019
(73) Proprietor: Asahi Kasei Medical Co., Ltd., Tokyo 100-0006 (JP)
(72) Inventor: OKAZAKI, Soichiro, Tokyo 101-8101 (JP); KAWASHIMA, Masato, Tokyo 101-8101 (JP)
(74) Representative: dompatent von Kreisler Selting Werner - Partnerschaft von Patent- und Rechtsanwälten mbB
(86) International application number: PCT/JP2017/013770
(87) International publication number: WO 2017/171064

(56) References cited:
- EP-A1- 2 735 325
- EP-A1- 2 735 325
- EP-A2- 1 684 825
- WO-A1-2015/152236
- JP-A- 2011 110 098
- US-A1- 2003 010 718
- US-A1- 2010 096 311
- US-A1- 2010 274 172
- US-A1- 2011 208 105
- US-A1- 2014 074 008
- US-B2- 9 220 830

## Description

### FIELD

The present invention relates to a blood purification system and a method for priming the same.

### BACKGROUND

Blood purification systems are widely known that perform purification treatment on blood using a blood purifier that purifies blood circulating outside the body (to also be referred to as "extracorporeal blood").

Blood purifiers typically have a first space and second space partitioned by a blood purification membrane, wherein blood flows into the first space and a fluid that purifies the blood, such as dialysate, flows into the second space.

A so-called priming procedure is carried out prior to performing blood purification treatment by which the blood purification device is made ready to initiate purification treatment by flushing out and removing fine particles, membrane protective agent, filling fluid and air present in the blood purification device and blood circuit with a priming fluid.

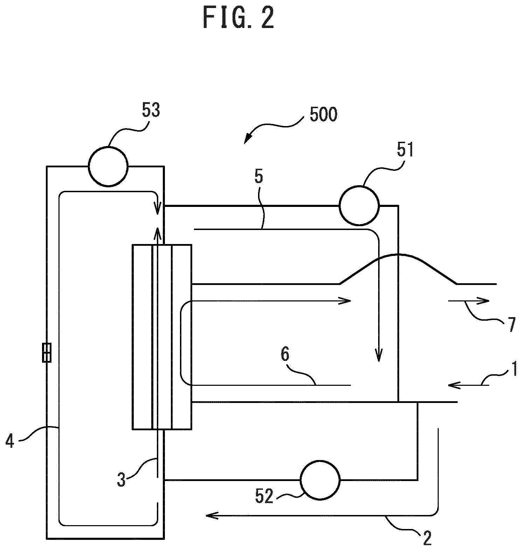

For example, PTL1 (Japanese Unexamined Patent Publication No. 2011-110098) describes a blood purification system applied to online hemodiafiltration having a dialysate supply line L6 connected to an arterial blood circuit 2, a first connecting line L4 connected to a chamber 9 of the arterial blood circuit 2, and a second connecting line L5 connected to a chamber 10 of a venous blood circuit 3. In the blood purification system of PTL1, the dialysate supply line L 6 supplies dialysate to the arteri al blood circuit 2 and the fi rst connecti ng line L 4 and the second connecti ng line L5 discharge dialysate through a branch line L3 during priming (refer to Figs. 2, etc., of PTL1).
EP 1684825, EP 2735325 and US 9220830 disclose a blood treatment system according to the preamble of claim 1.

### [CITATION LIST]

### [PATENT LITERATURE]

[PTL1] Japanese Unexamined Patent Publication No. 2011-110098

### SUMMARY

### [TECHNICAL PROBLEM]

Accompanying the recent increase in the number of patients requiring blood purification, the number of patients at a time that have to be managed by a single supervisi ng nurse or physician is increasing, thereby resulting in an increase in their workload. For example, during actual blood purification treatment, a single supervising nurse or physician may manage ten or more patients at a time. At this time, for the sake of simplification, if there were assumed to be ten checkpoints (although there are actually thought to be more) involving connection and disconnection of the blood circuit and so forth that must be checked for each blood purification treatment, this means that these confirmation procedures would be requi red to be performed more than 100 times in order to manage 10 or more patients. Thus, there is a strong desire to simplify blood purification treatment and reduce the workload on health care personnel. If it were possible to reduce the workload in this manner, this would not only lead to greater work efficiency but also prevention of mistakes.

One of the tasks associated with the greatest workload is the pri mi ng procedure carried out prior to blood purification treatment. Although the priming procedure performed for conventional blood purification systems varies according to such factors as the blood purifier used or structure of the blood circuit, since pri mi ng of the blood circuit and priming of the dialysate circuit are typically performed independently, it was necessary for the worker to change connections of circuits such as the blood circuit as well as change the settings of the pump and device each time priming was performed. For example, in the blood purification system of the prior art described in PTL1, when filling the blood circuit with dialysate, the first connecting line L4 and the second connecting line L5 are used to discharge dialysate from the blood circuit, and since solenoid valves V1, V2 and V7 are closed, priming of the flow path and dialysate circuit between dialysate inlet ports 1c and 1d are carried out in separate steps (Fig. 2 of PTL1). At this time, it is necessary to confi rm whether or not the blood circuit is properly and adequately replaced with dialysate followed by switchi ng over to the pri mi ng of the dialysate circuit, thereby placing a considerable burden on the worker.

In addition, since the type of hemodialyzer used differs accordi ng to the health care setti ng and the manner in which pri mi ng fluid is allowed to flow through the system may vary corresponding to the escape of air and ultrafiltration rate (UFR) inside and outside the blood purification membrane of the hemodialyzer, there is a desire for a blood purification system that offers a high degree of universality.

An object of the present invention is to provide a blood purification system that is able to reduce the workload associated with the priming procedure naturally required to be carried out during conventional blood purification treatment as previously described and thereby contribute to prevention of mistakes while also having superior universality, and to provide a method for priming that blood purification system.

### [SOLUTION TO PROBLEM]

As a result of conducting extensive studies to solve the aforementioned problems, the inventors of the present invention found that the aforementioned problems can be solved by employing a blood purification system having a specific circuit structure and fluid transfer means, thereby leading to completion of the present invention. Namely, the present invention is as described below:
[1] An online blood purification system comprising:
   a blood purifier that comprises a first space and second space partitioned by a blood purification membrane and purifies blood by passing extracorporeal blood through the first space,
   a blood inlet side flow path through which the extracorporeal blood flows prior to flowing into the first space,
   a blood outlet side flow path through which the extracorporeal blood flows after having flown out from the first space,
   a fluid supply flow path for supplying a fluid to the second space, and
   a fluid recovery flow path for recovering fluid from the second space, characterized in that the online blood purification system further comprises:
   an arterial side replacement fluid supply flow path that branches from the fluid supply flow path and is connected to the blood inlet side flow path, and
   a venous side replacement fluid supply flow path that branches from the fluid supply flow path and is connected to the blood outlet side flow path;
   wherein the arterial side replacement fluid supply flow path and the venous side replacement fluid supply flow path respectively comprise a fluid transfer means that can be independently controlled,
   wherein the blood inlet side flow path and the blood outlet side flow path are interconnectable, and
   wherein residual blood remaining in the first space can be recovered by closing either the arterial side replacement fluid supply flow path or the venous side replacement fluid supply flow path and allowing fluid for recovering blood to flow into the blood inlet side flow path or the blood outlet side flow path from the other replacement fluid supply flow path that is not closed.
[2] The blood purification system according to [1], wherein the fluid supply flow path is a dialysate supply flow path for supplying dialysate to the second space,
   the fluid recovery flow path is a dialysate recovery flow path for recovering dialysate from the second space, and
   the blood purifier is a hemodialyzer capable of purifying blood by contacting the extracorporeal blood with the dialysate.
[3] The blood purification system according to [2], wherein the blood purifier is a hollow fiber hemodialyzer that purifies blood by contacting dialysate flowing over the outside of hollow fibers with extracorporeal blood flowing inside the hollow fibers.
[4] The blood purification system according to any of [1] to [3], wherein the blood purification system comprises a reusable blood purification device,
   the blood purification device comprises the fluid supply flow path, the fluid recovery flow path, the arterial side replacement fluid supply flow path and the venous side replacement fluid supply flow path,
   the arterial side replacement fluid supply flow path and the venous side replacement fluid supply flow path supply replacement fluid directly to the extracorporeal blood, and
   the blood purification device further comprises a replacement fluid pressure measuring means for detecting abnormalities in the flow of the extracorporeal blood.
[5] The blood purification system according to [4], wherein the replacement fluid pressure measuring means directly measures the pressure of replacement fluid flowing through a region in communication with the arterial side replacement fluid supply flow path and/or the venous side replacement fluid supply flow path.
[6] The blood purification system according to any of [1] to [5], wherein the blood purifier comprises an arterial side end portion having a blood inlet and a venous side end portion having a blood outlet, the blood inlet side flow path is connected to the blood inlet and the blood outlet side flow path is connected to the blood outlet, and
   the further comprises a replacement fluid inlet in the arterial side end portion and/or the venous side end portion, and the arterial side replacement fluid supply flow path and/or the venous side replacement fluid supply flow path are connected to the replacement fluid inlet.
[7] The blood purification system according to [6], further comprising a mixing means in the arterial side end portion and/or the venous side end portion for uniformly mixing the extracorporeal blood and replacement fluid flowing in from the replacement fluid inlet.
[8] The blood purification system according to any of [1] to [7], wherein the blood purifier comprises an arterial side end portion comprising a blood inlet and a venous side end portion comprising a blood outlet, the blood inlet side flow path is connected to the blood inlet, and the blood outlet side flow path is connected to the blood outlet, and
   the blood purifier further comprises an internal space of a prescribed volume in the arterial side end portion and/or the venous side end portion that is capable of capturing gas mixed in or generated during blood purification.
[9] The blood purification system according to any of [1] to [8], composed of at least one tubular member selected form the group consisting of the fluid supply flow path, the fluid recovery flow path, the arterial side replacement fluid supply flow path, the venous side replacement fluid supply flow path, the blood inlet side flow path and the blood outlet side flow path.
[10] The blood purification system according to any of [1] to [9], wherein the fluid supply flow path and the fluid recovery flow path are interconnectable.
[11] The blood purification system according to any of [1] to [10], wherein the arterial side replacement fluid supply flow path and the venous side replacement fluid supply flow path are interconnectable.
[12] The blood purification system according to any of [1] to [11], wherein, in the fluid supply flow path,
   the junction of the arterial side replacement fluid supply flow path and the fluid supply flow path is located downstream from the junction of the venous side replacement fluid supply flow path and the fluid supply flow path.
[13] The blood purification system according to any of [1] to [12], wherein the junction of the arterial side replacement fluid supply flow path and the fluid supply flow path is located upstream from the junction of the venous side replacement fluid supply flow path and the fluid supply flow path.
[14] A method for priming the blood purification system according to any of [1] to [13], comprising:
   recovering priming fluid from the fluid recovery flow path through the second space of the blood purifier, by allowing the priming fluid to flow into the fluid supply flow path, and
   returning the priming fluid flowing through the fluid supply flow path to the fluid supply flow path through the first space of the blood purifier and the arterial side replacement fluid supply flow path, by allowing to flow into the venous side replacement fluid supply flow path.
[15] A method for priming the blood purification system according to any of [1] to [13], comprising:
   recovering priming fluid from the fluid recovery flow path through the second space of the blood purifier, by allowing the priming fluid to flow into the fluid supply flow path, and
   returning the priming fluid flowing through the fluid supply flow path to the fluid supply flow path through the first space of the blood purifier and the venous side replacement fluid supply flow path, by allowing to flow into the arterial side replacement fluid supply flow path.
[16] A method for priming the blood purification system according to any of [1] to [13], comprising:
   allowing priming fluid to flow into the fluid supply flow path,
   recovering the priming fluid from the fluid recovery flow path through the second space of the blood purifier, and
   returning the priming fluid passing through the second space to the fluid supply flow path through the arterial side replacement fluid supply flow path and/or the venous side replacement fluid supply flow path, by allowing to pass through the blood purification membranes and allowing to flow into the first space.
[17] A method for priming the blood purification system according to any of [1] to [13], comprising:
   allowing priming fluid to flow into the fluid supply flow path and allowing the priming fluid flowing through the fluid supply flow path to flow into the first space of the blood purifier through the arterial side replacement fluid supply flow path and/or the venous side replacement fluid supply flow path, and
   recovering the priming fluid flowing through the first space from the fluid recovery flow path by passing through the blood purification membrane and allowing to flow into the second space.

Examples of embodiments of a blood purification device suitable for use in the blood purification system and priming method thereof of the present embodiment are as indicated below.
[20] A blood purification device used with a disposable blood purifier for purifying extracorporeal blood,
   wherein the blood purification device comprises at least one replacement fluid supply flow path for directly supplying replacement fluid to the extracorporeal blood and a replacement fluid pressure measuri ng means that measures the pressure of replacement fluid flowing through the replacement fluid supply flow path for detecting abnormalities in the flow of the extracorporeal blood,
   the replacement fluid supply flow path consists of two systems, one system for supplying replacement fluid to the extracorporeal blood and the other system for supplying replacement fluid to the extracorporeal blood following purification, and
   the replacement fluid supply flow paths of the two systems respectively comprise a fluid transfer means that can be independently controlled.
[19] The blood purification device described in [18], wherein the blood purifier is a hemodialyzer that purifies blood by contacting dialysate with the extracorporeal blood, and
   the blood purification device further comprises a dialysate supply flow path for supplying dialysate to the hemodialyzer and a dialysate recovery flow path for recoveri ng dialysate from the hemodialyzer.
[20] The blood purification device described in [19], wherein the hemodialyzer is a hollow fiber hemodialyzer that purifies blood by contacting dialysate flowing over the outside of hollow fi bers with extracorporeal blood flowing through the inside of the hollow fi bers.
[21] The blood purification device described in [19] or [20], wherein the replacement fluid supply flow path branches from the dialysate supply flow path and supplies dialysate to the extracorporeal blood as replacement fluid.
[22] The blood purifi cati on device descri bed in any of [18] to [21], wherein the blood purifier comprises an arterial side end portion comprising a blood inlet and a venous side end portion comprising a blood outlet, and
   the blood purifier further comprises a replacement fluid inlet in the arterial side end porti on and/or the venous side end porti on, the replacement fluid inlet is connected to the replacement fluid supply flow path of the blood purifi cati on device at the time of use allowing replacement fluid to flow there through.
[23] The blood purification device described in [22], wherein the blood purifier further comprises a mixing means in the arterial side end portion and/or the venous side end portion of the blood purifier for uniformly mixing the extracorporeal blood and replacement fluid flowing in from the replacement fluid inlet.
[24] The blood purification device described in [22] or [23], wherein the blood purifier comprises an internal space of a prescribed volume in the arterial side end portion and/or the venous side end portion of the blood purifier capable of capturing gas mixed in or generated during blood purification.
[25] The blood purifi cati on device descri bed in any of [20] to [24], wherein the blood purifier further comprises a replacement fluid inlet tubular member connected to the replacement fluid inlet, and the replacement fluid inlet tubular member is connected to the replacement fluid supply flow path of the blood purifi cati on device at the time of use allowing replacement fluid to flow there through.

### [ADVANTAGEOUS EFFECTS OF INVENTION]

Since the blood purification system and method for priming the same of the present invention have the configurations descri bed above, the workload associated with priming can be reduced, thereby contributing to prevention of mistakes and demonstrating superior universality.

### BRIEF DESCRIPTION OF DRAWINGS

FIG. 1 is a schematic diagram of a blood purification system according to an embodiment of the present invention.
FIG. 2 is a schematic diagram showing a first embodiment of a method for priming a blood purification system according to an embodiment of the present invention.
FIG. 3 is a schematic diagram showing a second embodiment of a method for priming a blood purification system according to an embodiment of the present invention.
FIG. 4 is a schematic diagram showing a third embodiment of a method for priming a blood purification system according to an embodiment of the present invention.
FIG. 5 is a schematic diagram showing a fourth embodiment of a method for priming a blood purification system according to an embodiment of the present invention.
FIG. 6 is a schematic diagram exemplifying an aspect of blood recovery by a blood purification system accordi ng to an embodi ment of the present invention.
FIG. 7 is a schematic diagram of a blood purifier and blood purification device used in a preferred embodi ment of the blood purification system of the present invention.
FIG. 8 is a schematic diagram showing a cross-section of a blood purifier used in a preferred embodi ment of the blood purification system of the present invention.
FIG. 9 is a schematic diagram showing a cross-section of a blood purifier.

### DESCRIPTION OF EMBODIMENTS

Although the following provides a detailed explanation of a blood purifi cati on system and method for pri mi ng the same accordi ng to embodiments of the present invention (to be referred to as a "present embodi ment") with reference to the drawings.

<<Blood Purification System>

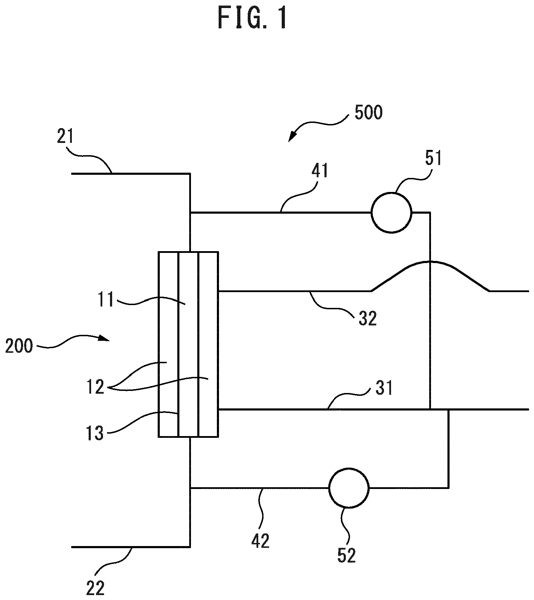

Fig. 1 is a schematic diagram showing a blood purification system according to the present embodiment. The blood purification system (500) according to the present embodiment is an online blood purification system having: a blood purifier (200) having a firstspace(11) and second space (12) partitioned by blood purification membrane (13) that purifies blood through extracorporeal blood in the first space, a blood inlet side flow path (21) through which extracorporeal blood flows prior to flowing into the fi rst space, a blood outlet side flow path (22) through which extracorporeal blood flows after having flown out from the first space, a blood supply flow path (31) for supplying fluid to the second space, a fluid recovery flow path (32) for recovering fluid from the second space, an arterial side replacement fluid supply flow path (41) branched from the fluid supply flow path and connected to the blood inlet side flow path, and a venous side replacement fluid supply flow path (42) branched from the fluid supply flow path and connected to the blood outlet side flow path. The arterial side replacement fluid supply flow path and the venous side replacement fluid supply flow path respectively have a fluid transfer means (51 and 52) that can be independently controlled.

As a result of having the aforementioned configuration, since the blood purification system of the present embodi ment is able to allow pri mi ng fluid that has passed through the first space of the blood purifier to flow into the second space without discarding or is able to allow priming fluid that has flown into the second space to flow into the first space without discarding, the tasks of reconnecting circuits or switching pumps are no longer required, thereby allowing pri mi ng of the first space and second space to be carried out si multaneously and reducing the workload.

Moreover, the blood purification system of the present embodiment can be used for any of hemodialysis treatment (typically abbreviated as "HD"), hemodiafiltration treatment (typically abbreviated as "HDF") and hemofiltration treatment (typically abbreviated as "HF") as will be subsequently described. In addition, since the blood purification system of the present invention has two systems consisti ng of an arterial side replacement fluid supply flow path and a venous side replacement supply flow path for the flow paths capable of supplying replacement fluid to the blood circuit, and the flow paths respectively have a fluid transfer means that can be independently controlled, replacement fluid can be supplied or recovered from either system. Thus, a suitable flow can be set during the priming procedure that corresponds to the type of hemodialyzer. During hemodialysis treatment, replacement fluid can be supplied upstream from the first space of the blood purifier (also referred to as "pre-dilution"), replacement fluid can be supplied downstream from the first space (also referred to as "post-dilution"), or replacement fluid can simultaneously be supplied upstream and downstream from the first space (also referred to as "simultaneous pre-post-dilution"). During recovery of blood, blood remaining in the blood circuit can be recovered by supplying fluid for recovering blood from one of the arterial side replacement fluid supply flow path or venous side replacement fluid supply flow path, thereby reducing the workload associated with the blood recovery procedure and further contributing to prevention of mistakes. Thus, the blood purification system of the present invention reduces the workload associated with priming, thereby contributing to safety and demonstrating superior universality.

### <Blood Purifier>

The blood purifier in the blood purification system of the present embodiment has a first space and second space partitioned by a blood purification membrane, and there are no particular limitations thereon provided blood can be purified by passing extracorporeal blood through the fi rst space.

The types of blood purifiers can generally be broadly classified according to the principle used to purify blood. Examples of principles used to purify blood include filtration, dialysis and combinations thereof, and in the present embodiment, the blood purifier may be of any type.

The blood purifier may be hemofilter that purifies blood by filtering extracorporeal blood with a blood purification membrane. Since this type of hemofilter can be used as a portion of the blood purification system of the present embodiment, the present invention does not preclude the use of these types of hemofilters.

The blood purifier may also be a hemodialyzerthat purifies blood by contacting a dialysate with extracorporeal blood through a blood purification membrane. The blood purifier may be a so-called hollow fiber hemodialyzer that uses hollow fibers for the blood purification membrane and purifies blood by allowing dialysate flowing over the outside of the hollow fibers to contact extracorporeal blood flowing through the inside of the hollow fibers.

The hemodialyzer is able to purify blood by using the principle of diffusion or combi ni ng diffusion and filtration. In the description of the present application, the term "hemodialyzer" includes a so-called "hemodiafilter" that purifies blood by combining diffusion with filtration.

The blood purifier typically has an arterial side end portion having a blood inlet and a venous side end porti on having a blood outlet in order to allow extracorporeal blood to pass there through. Here, in the description of the present application, an "arterial side end portion" refers to a portion of the blood purifier located farther upstream than the portion substantially having the function of purifying extracorporeal blood (to also be referred to as the "blood purification unit" represented by reference symbol 216 in the drawings), while the "venous side end portion" refers to the portion of the blood purifier located farther downstream than the blood purification unit. The arterial side end portion and the venous side end portion may be separation components from the main body receptacle such as in the form of covers, or may be components integrally formed with the main body receptacle. There are no particular limitations on the shape of the main body receptacle of the blood purifier and may have, for example, a tubular shape and typically a cylindrical shape.

### <Blood Inlet Side Flow Path and Blood Outlet Side Flow Path>

T he blood purification system of the present embodi ment has a blood inlet side flow path through which extracorporeal blood flows prior to flowing into the first space of the blood purifier, and a blood outlet side flow path through which extracorporeal blood flows after having flown out from the first space. Furthermore, in the descri pti on of the present appl i cati on, the term "blood inlet side flow path" refers to a flow path that includes the internal space of the arterial side end portion of the blood purifier and extends to immediately before the extracorporeal blood flows into the first space, while the term "blood outlet side flow path" refers to a flow path that includes the internal space of the venous side end porti on of the blood purifi er and extends downstream from i mmedi ately after the extracorporeal blood has flown out from the first space.

In the present embodi ment, the blood purifier may have an arterial side end portion having a blood inlet and a venous side end portion having a blood outlet, the blood inlet side flow path may be connected to the blood inlet and the blood outlet side flow path may be connected to the blood outlet. At least one of the blood inlet si de flow path and the blood outlet side flow path is a tubular member, and for example, a blood inlet tubular member may be connected to the blood inlet to compose the blood inlet side flow path, and a blood outlet tubular member may be connected to the blood outlet to compose the blood outlet side flow path. At the ti me of use, the blood inlet si de flow path, the first space and the blood outlet si de flow path communicate to compose a blood circuit through which extracorporeal blood is able to flow.

In the blood purification system of the present embodi ment, the blood inlet side flow path and the blood outlet side flow path are interconnectable. Since i nterconnecti on of the blood inlet side flow path and the blood outlet side flow path makes it possible to circulate priming fluid through the blood circuit by connecting these flow paths during priming, the priming procedure can be carried out more efficiently. There are no particular limitations on the interconnection mode between the blood inlet side flow path and the blood outlet side flow path.

The blood inlet side flow path may also have a blood pump. The fluid transfer means of the arterial side replacement fluid supply flow path, the fluid transfer means of the venous side replacement fluid supply flow path, and the blood pump of the blood inlet side flow path may each be able to be independently controlled.

### <Fluid Supply Flow Path and Fluid Recovery Flow Path>

The blood purification system of the present embodiment has a fluid supply flow path for supplying fluid to the second space of the blood purifier and a fluid recovery flow path for recovering fluid from the second space. Thus, the blood purifi cati on system of the present embodiment can be used for any blood purification treatment that purifies blood by utilizing the principle of diffusion and using a dialysate, examples of which include hemodialysis treatment (typically abbreviated as "HD") and hemodiafiltration treatment (typically abbreviated as "HDF"). In the description of the present application, blood purification treatment utilizing the principle of diffusion, and including HD and HDF, is collectively referred to as "hemodialysis treatment".

In the present embodiment, in the case the blood purifier is a hemodialyzer capable of purifying blood by contacting extracorporeal blood with the aforementioned dialysate, the fluid supply flow path is a dialysate supply flow path for supplying dialysate to the second space, and the fluid recovery flow path is a dialysate recovery flow path for recovering dialysate from the second space.

There are no particular li mitations on the dialysate provided it is able to remove excess moisture and waste products from blood by contacting with extracorporeal blood. One example of a dialysate is physiological saline. Dialysate may also be used as replacement fluid. There are no limitations on the means for transferring the dialysate (not shown), and an arbitrary fluid transfer pump, such as a duplex pump or tubing pump, can be used.

At least one of the fluid supply flow path and fluid recovery flow path may be composed of a tubular member.

The fluid supply flow path and the fluid recovery flow path are able to be interconnected. When cleaning and disinfecting the inside of the flow paths of the blood purification device, since interconnecti ng the fluid supply flow path and fluid recovery flow path makes it possible to circulate the cleaning disinfectant there through, cleani ng and disinfection of the flow paths can be carried out efficiently. There are no particular limitations on the interconnection mode between the fluid supply flow path and the fluid recovery flow path.

### <Arterial Side Replacement Fluid Supply Flow Path and Venous Side Replacement Fluid Supply Flow Path>

The blood purification system of the present embodiment has an arterial side replacement fluid supply flow path that branches from the fluid supply flow path and is connected to the blood inlet side flow path, and a venous side replacement fluid supply flow path that branches from the fluid supply flow path and is connected to the blood outlet side flow path.

During blood purification treatment, since adjustment of material balance with respect to moisture and electrolytes in the blood as well as adjustment of pH are typically carried out accompanying removal of moisture and waste products from the blood, replacement fluid is supplied to the extracorporeal blood. In the present embodiment, the blood purification system is able to supply replacement fluid to extracorporeal blood through the arterial side replacement fluid supply flow path and/or venous side replacement fluid supply flow path.

At least one of the arterial side replacement fluid supply flow path and venous side replacement fluid supply flow path may be composed of a tubular member.

There are no particular limitations on the location where the arterial side replacement fluid supply flow path is connected to the blood inlet side flow path, and may be connected upstream from the blood purifier, to a drip chamber, or to a replacement fluid inlet provided in the arterial side end portion of the blood purifier. There are also no limitations on the location where the venous side replacement fluid supply flow path is connected to the blood outlet side flow path, and may be connected downstream from the blood purifier, to a drip chamber or to a replacement fluid inlet provided in the venous side end portion of the blood purifier.

There are no particular limitations on the positional relationship of the junction between the arterial side replacement fluid supply flow path and the fluid supply flow path or on the positional relationship of the junction between the venous side replacement fluid supply flow path and the fluid supply flow path. For example, the junction between the arterial side replacement fluid supply flow path and the fluid supply flow path may be downstream or upstream from the junction between the venous side replacement fluid supply flow path and the fluid supply flow path.

The arteri al side replacement fluid supply flow path and the venous side replacement fluid supply flow path are able to be interconnected. When cleaning and disinfecting the inside of the flow paths of the blood purification device, since interconnecting the arterial side replacement fluid supply flow path and venous side replacement fluid supply flow path makes it possible to circulate the cleaning disinfectant there through, cleani ng and disinfection of the flow paths can be carried out efficiently. The fluid supply flow path and the fluid recovery flow path may be able to be interconnected and the arterial side replacement fluid supply flow path and the venous side replacement fluid supply flow path may be able to be interconnected. In this case, the inside of the flow paths of the blood purification device can be cleaned and disinfected even more efficiently. There are no particular limitations on the interconnection mode between the arterial side replacement fluid supply flow path and the venous side replacement fluid supply flow path.

### <Replacement Fluid Inlet>

In the present embodiment, the blood purifier further has a replacement fluid inlet in the arterial side end portion and/or venous side end portion, and the arterial side replacement fluid supply flow path and/or venous side replacement fluid supply flow path may be connected to the replacement fluid inlet.

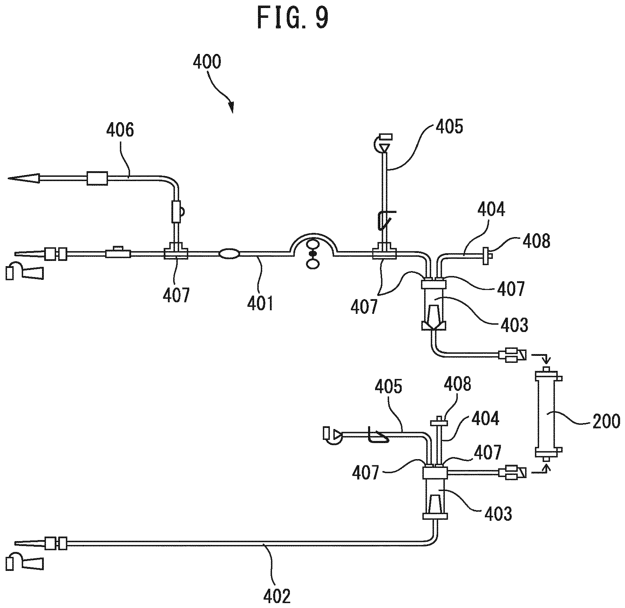

It was conventionally necessary to supply replacement fluid from replacement fluid inlet tubular members (405) provided on blood inlet tubular member and drip chamber as shown in Fig. 9. Thus, the replacement inlet fluid tubular member per se as well as the accompanyi ng connecting members (407) were also conventionally necessary. Since the replacement member tubular members (405) and the connecting members (407) thereof shown in Fig. 9 are no longer required by connecting the arterial side replacement fluid supply flow path and/or venous side replacement fluid supply flow path to the replacement fluid inlet of the arterial side end portion and/or venous side end portion, the blood circuit is simplified, thereby contributing to prevention of mistakes. In addition, production costs are reduced due to the use of fewer parts, thereby making it possible to reduce the number of members discarded following each blood purification treatment and demonstrate superior economy.

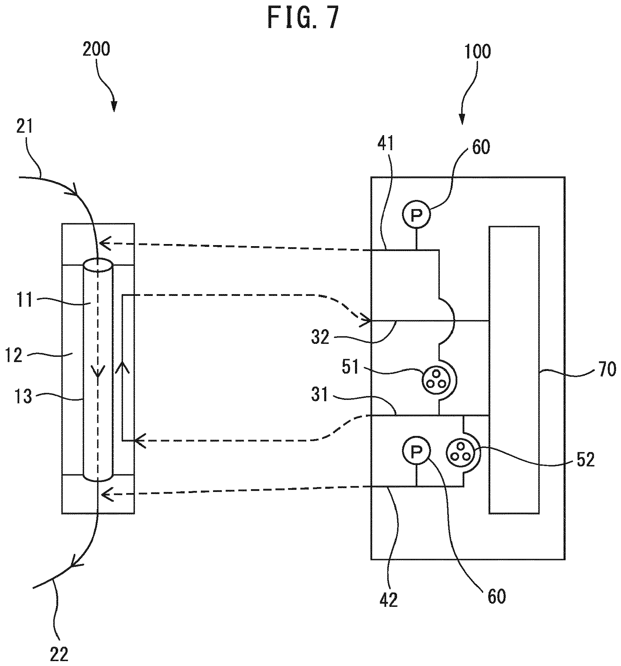

In the case of an online blood purification system, the replacement fluid and/or dialysate are typically supplied to the blood purification system after having been prepared at a location such as hospital where blood purification is carried out by mixing undiluted replacement fluid and/or dialysate with water having a high degree of cleanli ness. Preparation and supply of the replacement fluid and/or dialysate may be carried out with a replacement fluid (dialysate) supply source. The replacement fluid (dialysate) supply source may be supply source (70) provided within the blood purification device, as shown in Fig. 7, for example, or may be a supply source provided separately from the blood purification device such as a replacement fluid (dialysate) supply device (not shown). There are no limitations on the fluid transfer means for transferring replacement fluid and/or dialysate (not shown in Fig. 1), and any type of fluid transfer pump, such as a duplex pump or tubing pump, can be used.

### (Mixing Means)

In the present embodiment, the blood purifier further has a replacement fluid inlet in the arterial side end portion and/or venous side end portion, the arterial side replacement fluid supply flow path and/or the venous side replacement fluid supply flow path are connected to the replacement fluid inlet, and the blood purifier may further have a mixing means in the arterial side end portion and/or venous side end portion for uniformly mixing extracorporeal blood with replacement fluid flowing in from the replacement fluid inlet.

As a result of the blood purifier having a mixing means in the arterial side end portion, mixing of extracorporeal blood and replacement fluid is promoted, blood purification can be carried out more uniformly, and this can be expected to demonstrate the effect of reducing decreases in function of the blood purifi er. As a result of having a mixing means in the venous side end portion, mixing of extracorporeal blood and replacement fluid is promoted and the burden on the patient can be reduced.

There are no particular limitations on the mixing means and any suitable means can be selected by a person with ordinary skill in the art. For example, a mixing means not having a drive unit is preferable for the mixing means from the viewpoints of safety and ease of production. Examples of mixing means not having a drive unit include those provided with obstructions such as baffle plates or projections, and those provided with a structure typically referred to as a static mixer. Furthermore, this mixing means does not preclude aspects in which the shape of the replacement fluid inlet and the shape of the aforementioned end porti ons collectively act together to mix blood and replacement fluid.

### <Internal Space>

In the present embodiment, the blood purifier has an arterial side end portion having a blood inlet and a venous side end portion having a blood outlet, a blood inlet side flow path is connected to the blood inlet, a blood outlet side flow path is connected to the blood outlet, and the blood purifier may also have an internal space of a prescribed volume in the arterial side end portion and/or venous side end portion for capturing gas mixed in or generated during blood purification.

It was conventionally necessary to capture gas mixed in or generated during blood purification by providing a drip chamber (403) on the blood inlet tubular member and/or blood outlet tubular member so as to prevent entry of gas into the body of a patient as shown in Fig. 9. The drip chamber on the blood outlet tubular member was particularly important since blood returni ng to the body of the patient flows through the blood outlet tubular member. In contrast, in the present embodiment, as a result of the arterial side end portion and/or venous side end portion of the blood purifier having an internal space as previously described, it is no longer necessary to provide the conventionally required drip chamber shown in Fig. 9, thereby simplifying the blood circuit, making it possible to reduce the workload and further contributing to prevention of mistakes, while also reducing contact between the blood and air and effectively reducing blood coagulation. Moreover, production costs are reduced due to fewer parts, thereby making it possible to reduce the number of members discarded following each blood purification treatment and demonstrate superior economy.

Although there are no particular limitations thereon, the prescribed volume of the internal space can be, for example, 5 cm³ to 30 cm³.

### <Drug Introduction Port>

In the present invention, a drug introduction port may be further provided in the arterial side end porti on of the blood purifier. It is necessary to introduce an anticoagulant in order to prevent coagulation of extracorporeal blood due to contact between the blood and air during blood purification treatment. In the past, a drug introduction port (not shown) was provided in a component such as the blood inlet tubular member. In contrast, in the present embodi ment, in the case of further having a drug introduction port in the arteri al side end porti on of the blood purifier, it is no longer necessary to provide a conventional drug introduction port, thereby simplifying the blood circuit and contribution to prevention of mistakes.

### < Fluid Transfer Means>

The blood purification system of the present embodiment respectively has a fluid transfer means in the arterial side replacement fluid supply flow path and venous side replacement fluid supply flow path that can be independently controlled.

These independently controllable fluid transfer means make it possible to arbitrarily set closing and opening of the arterial side replacement fluid supply flow path and venous side replacement fluid supply flow path, fluid transfer as well as the flow rate and direction during fluid transfer, while also allowing the settings to be changed at arbitrary times.

The independently controllable fluid transfer means may be made able to change closing and opening of the arterial side replacement fluid supply flow path and venous side replacement fluid supply flow path, fluid transfer as well as the flow rate and direction during fluid transfer based on various parameters relating to use of the blood purification system, such as the pressure, temperature and flow rate of the extracorporeal blood, dialysate and/or replacement fluid.

There are no particular limitations on the fluid transfer means and an arbitrary fluid transfer pump, such as a duplex pump or tubing pump, can be used.

### <Blood Purification Device>

The blood purification system of the present embodiment can be used with a reusable blood purification device. The blood purification device may have a fluid supply flow path, fluid recovery flow path, arterial side replacement fluid supply flow path and venous side replacement fluid supply flow path. The arterial side replacement fluid supply flow path and the venous side replacement fluid supply flow path are able to directly supply replacement fluid to extracorporeal blood, and the blood purification device may further have a replacement fluid pressure measuri ng means for detecting abnormalities in the flow of the extracorporeal blood. The replacement fluid pressure measuring means may be a replacement fluid pressure measuring means that directly measures the pressure of replacement fluid flowing through a region in communication with the arterial side replacement fluid supply flow path and/or venous side replacement fluid supply flow path. In the description of the present application, the "region in communication with the arterial side replacement fluid supply flow path and/or venous side replacement fluid supply flow path" refers to a region where abnormalities in the flow of extracorporeal blood can be detected by measuri ng the pressure of the replacement fluid with the replacement fluid pressure measuring means. In other words, this communicating region can be said to be a region having a fluid pressure that correl ates with the fluid pressure of the extracorporeal blood. Thus, it should be noted that, for example, a region located upstream from the fluid transfer means of the arterial side replacement fluid supply flow path and venous side replacement fluid supply flow path is not a "region in communication with the arterial side replacement fluid supply flow path and/or venous side replacement fluid supply flow path" since abnormalities in the flow of extracorporeal blood cannot be detected in this region even if a replacement fluid pressure measuring means is provided, but rather the portion located downstream from the fluid transfer means of the arterial side replacement fluid supply flow path and venous side replacement fluid supply flow path is applicable to the "region in communication with the arterial side replacement fluid supply flow path and/or venous side replacement fluid supply flow path".

As shown in Fig. 9, a tubular member for measuring pressure (404) was connected to a drip chamber (403) provided in the blood circuit in order to detect abnormalities in blood flow in the prior art. This tubular member for measuring pressure is connected to the upper portion of a drip chamber so as not to contact blood and passes through a gaseous phase inside the drip chamber. In the prior art, a pressure measuri ng device (not shown) was connected to the tubular member for measuring pressure and abnormalities in the flow of extracorporeal blood were detected via the gaseous phase within the drip chamber. Thus, the tubular member for measuri ng pressure per se as well as connecting members (407) for attaching the tubular member for measuring pressure were required in the prior art. In addition, procedures consisting of connecti ng the pressure measuri ng devi ce to the tubular member for measuri ng pressure pri or to blood purifi cati on treatment and removing the device following completion of blood purification treatment were required, thereby resulting in an increase in the workload.

In addition, the tubular member for measuring pressure (404) conventionally had a hydrophobic filter (408) to prevent microorganisms and other contaminants from entering the blood circuit from the pressure measuri ng means and to prevent leakage of blood from the blood circuit into the pressure measuring means. Since the tubular member for measuring pressure is no longer able to measure pressure within the drip chamber if the hydrophobic filter ends up getti ng wet, it was necessary to take into consideration measures for preventing the hydrophobic filter from becoming wet prior to blood purification treatment as well as monitor the hydrophobic filter during blood purifi cati on treatment to ensure that it did not become wet. However, there is the risk of the hydrophobic filter becoming wet with replacement fluid or blood present in the blood circuit as a result of air of the gaseous phase of the drip chamber becoming wet due to, for example, failing to properly connect the blood circuit. Once the hydrophobic filter has become wet, the blood circuit must be replaced with a new circuit, thereby resulting in an increase in the workload.

In contrast, as a result of the blood purification system containing a reusable blood purification device, the blood purification device having a fluid supply flow path, a fluid recovery flow path, an arterial side replacement fluid supply flow path and a venous side replacement fluid supply flow path, the arterial side replacement fluid supply flow path and the venous side replacement fluid supply flow path being able to directly supply replacement fluid to extracorporeal blood, and the blood purification device further having a replacement fluid pressure measuri ng means for detecting abnormalities in the flow of extracorporeal blood, the conventionally required tubular member for measuring pressure, the accompanying connecting members and the hydrophobic filter are no longer required, thereby simplifying the blood circuit. In addition, since the pressure a fluid in the form of the replacement fluid can be measured without having to go through the gaseous phase of a drip chamber, it is no longer necessary to provide a gaseous phase for measuring pressure within the drip chamber, thereby making it possible to reduce contact area between the gaseous phase and blood. Thus, the workload associated with connecting members prior to blood purification treatment and removing members following completion of blood purification treatment can be reduced, thereby contributing to prevention of mistakes, reducing contact between blood and air, reducing coagulation of blood and further reducing production costs due to the use of fewer parts, while also making it possible to reduce the number of members discarded following each blood purification treatment and demonstrate superior economy.

In the description of the present application, "being able to directly supply replacement fluid to extracorporeal blood" refers to composing so as to be able to detect abnormalities in the flow of extracorporeal blood by measuring the pressure of replacement fluid with a replacement fluid pressure measuring means. Thus, for example, a portion located upstream from the fluid transfer means of the arterial side replacement fluid supply flow path and venous side replacement fluid supply flow path is not a portion "able to directly supply replacement fluid to extracorporeal blood" since abnormalities in the flow of extracorporeal blood cannot be detected even if a replacement fluid pressure measuring means is provided, but rather the portion located downstream from the fluid transfer means is applicable to the portion "able to directly supply replacement fluid to extracorporeal blood".

There are no particular limitations on the location of the replacement fluid pressure measuring means provided it is in the arterial side replacement fluid supply flow path and venous sided replacement fluid supply flow path of the blood purification device that are capable of directly supplying replacement fluid to extracorporeal blood. In the case the arterial side replacement fluid supply flow path and/or venous side replacement fluid supply flow path for directly supplying replacement fluid to extracorporeal blood extend outside the case of the blood purification device, the replacement fluid pressure measuring means may also be installed outside the case of the blood purification device.

Any arbitrary pressure gauge can be used for the replacement fluid pressure measuring means provided it is able to measure the pressure of a fluid in the form of the replacement fluid. There are no particular limitations on the replacement fluid pressure measuring means, and examples thereof include elastic pressure gauges such as a Bourdon tube pressure gauge, diaphragm pressure gauge, bellows pressure gauge or chamber pressure gauge, and non-elastic pressure gauges such as a liquid column pressure gauge or dead weight type pressure gauge.

Whether or not there is an abnormality in the flow of extracorporeal blood can be suitably determined by a person with ordinary skill in the art in accordance with such as factors as patient status and blood purification treatment conditions. For example, the flow of blood can be determined to be abnormal when replacement fluid pressure has fluctuated from a steady state value by an amount equal to or greater than a prescribed value. The blood purification device may further have a control device that automatically determines whether or not there is an abnormality in the flow of extracorporeal blood. The control device may provide an indication for notifyi ng the operator when the flow of extracorporeal blood has been determined to be abnormal.

### <Blood Circuit>

The blood purification system of the present embodi ment is able to recover blood remaining within the first space by closing either of the arterial side replacement fluid supply flow path and venous side replacement fluid supply flow path and allowing fluid for recovering blood to flow into the blood inlet side flow path or blood outlet side flow path from the other replacement fluid supply flow path that is not closed. As a result, the blood recovery procedure can be initiated following completion of blood purification treatment without having to rearrange the blood circuit or replacement fluid circuit, thereby reducing the workload.

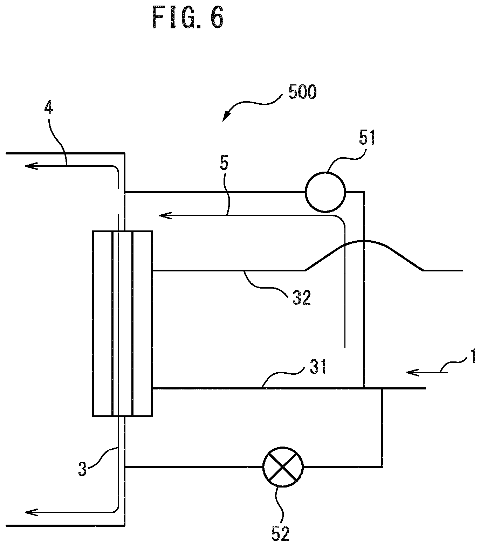

Fig. 6 is a schematic diagram exemplifying an aspect of a blood circuit according to the blood purification system according to an embodiment of the present invention. As shown in Fig. 6, by controlling the closed state of a fluid transfer means (52) of the venous side replacement fluid supply flow path, fluid for recovering blood is allowed to flow into the blood inlet side flow path from the arteri al side replacement fluid supply flow path by closing the venous side replacement fluid supply flow path and controlling a fluid transfer means (51) of the arterial side replacement fluid supply flow path (Flow 5), thereby making it possible to recover blood remai ni ng in the first space (Flow 3). Blood remai ni ng upstream from the first space can be recovered from arterial side paracentesis needle (Flow 4).

The blood purification system of the present embodi ment makes it possible to close the fluid supply flow path (31) and/or the fluid recovery flow path (32). There are no particular limitations on the closing means and examples thereof include a solenoid valve and clamp.

There are no particular limitations on the fluid for recovering blood provided it can be used to replace extracorporeal blood remai ni ng in the first space and blood circuit, and examples thereof that can be used include replacement fluid, dialysate and physiological saline. In the case of an online blood purification system, dialysate can be used for the fluid for recovering blood that can be used to replace extracorporeal blood remai ni ng in the first space and blood circuit. In the case of an online blood purification system, dialysate is also typically used as replacement fluid as was previously explained.

### <Preferred Embodiment of Blood Purification System>

Fig. 7 is a schematic diagram of a blood purifier and blood purification device used in a preferred embodi ment of the blood purification system of the present embodi ment. In Fig. 7, a blood purifier (200) is a hemodialyzer that purifies blood by contacting dialysate with extracorporeal blood and is used with a reusable blood purification device (100) to compose a blood purification system. The blood purification device (100) has an arterial side replacement supply flow path (41) connected to an arterial side end portion of the blood purifier and a venous side replacement fluid supply flow path (42) connected to a venous side end portion of the blood purifi er, and each of these di rectly supplies replacement fluid to extracorporeal blood flowing through a blood inlet side flow path (21) and a blood outlet side flow path (22). These blood supply flow paths each have fluid transfer means (51 and 52) that can be independently controlled. The blood purification device has a replacement fluid pressure measuring means (60) in each replacement fluid supply line that measures the pressure of replacement fluid flowing through the replacement fluid supply flow paths in order to detect abnormalities in the flow of extracorporeal blood. In addition, the blood purification device further has a dialysate supply flow path (31) for supplying dialysate to the hemodialyzer and a dialysate recovery flow path (32) for recovering fluid such as dialysate from the hemodialyzer. The two replacement fluid supply flow paths each branch from the dialysate supply flow path (31), and thus, the blood purification device (100) is an online blood purification device capable of supplying replacement fluid in the form of dialysate from a dialysate supply source (70) to extracorporeal blood.

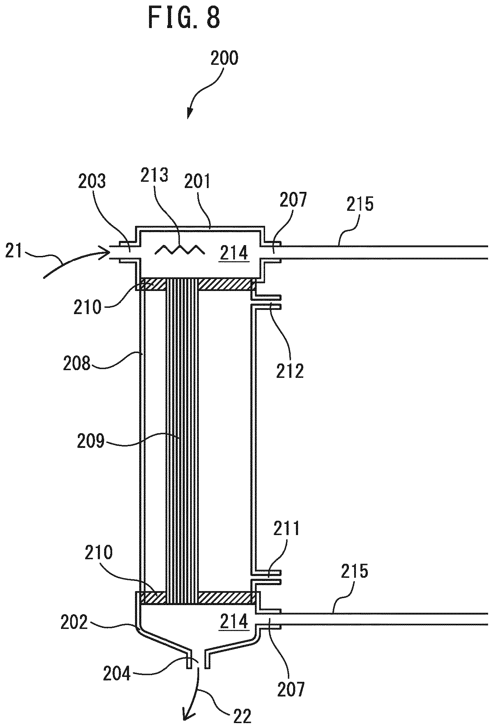

Fig. 8 is a schematic diagram showing a cross-section of a blood purifier used in a preferred embodi ment of the blood purification system of the present invention. In Fig. 8, a blood purifier (200) has a hollow fiber bundler (209) in a cylindrical main body receptacle (208) and both ends of the hollow fiber bundle is immobilized within the cylindrical main body receptacle by sealing members (210) to partition the inside and outside of the hollow fibers. Furthermore, only a portion of the hollow fiber bundle is depicted in the drawing for the sake of explanation.

The cylindrical main body receptacle has an arterial side end portion (201), having a blood inlet (203) and a replacement fluid inlet (207), and a venous side end porti on (202), having a blood outlet (204) and a replacement fluid outlet (207), on both ends thereof. At the time of use, a blood inlet tubular member (not shown) is connected to the blood inlet and a blood outlet tubular member (not shown) is connected to the blood outlet to compose of a blood circuit, thereby allowing extracorporeal blood to flow though the hollow fi bers at the ti me of use. In addition, replacement fluid inlet tubular members (215) are respectively connected to the replacement fluid inlet, and the replacement fluid inlet tubular members are connected to each of the two replacement fluid supply flow paths of the blood purification device (100) at the time of use to allow the flow of replacement fluid. The main body receptacle has a dialysate inlet (211) and a dialysate outlet (212) on the side thereof, and extracorporeal blood is allowed to contact dialysate via the hollow fibers by all owi ng dialysate to flow over the outside of the hollow fibers inside the main body receptacle through the space surrounded by the sealing members.

In addition, the blood purifier has a mixing means (213) within the arterial side end portion that is able to uniformly mix extracorporeal blood with replacement fluid flowing in from the replacement fluid inlet prior to blood passing through the i nsi de of the hollow fi bers. Moreover, the blood purifier has internal spaces (214) of a prescribed volume in the arterial side end portion and venous side end portion that is able to capture gas mixed in or generated duri ng blood purification.

### «Method for Priming Blood Purification System»

Although the following provides an explanation of an embodi ment of a method for pri mi ng the blood purification system of the present embodi ment with reference to the drawings, the method for priming the blood purification system is not limited to the present embodiment.

### < First Embodiment of Priming Method>

Fig. 2 is a schematic diagram showing a first embodiment of a method for priming the blood purification system accordi ng to the present embodi ment. The first embodiment of the pri mi ng system includes allowing priming fluid to flow into the fluid supply flow path from a priming fluid supply means (not shown) and recovering priming fluid from the fluid recover flow path through the second space of the blood purifier (Flows 1, 6 and 7), allowing a portion of the pri mi ng fluid flowing through the fluid supply flow path to flow into the venous side replacement fluid supply flow path (Flow 2), and returning the priming fluid to the fluid supply flow path through the first space of the blood purifier (Flow 3) and the arterial side replacement fluid supply flow path (Flow 5). As a result, the first space and the second space can be primed simultaneously. Priming fluid may be circulated (Flow 4) with a blood pump (53) by connecting the blood inlet side flow path and the blood outlet side flow path. The flow of pri mi ng fluid can be balanced at an arbitrary flow rate by independently controlling the fluid transfer means (52) of the venous side replacement fluid supply flow path, the fluid transfer means (51) of the arterial side replacement fluid supply flow path and the blood pump (53).

In the fi rst embodiment of the pri mi ng method, the inflow of priming fluid to the venous side replacement fluid supply flow path (Flow 2) and recovery of priming fluid from the arterial si de replacement fluid supply flow path (Flow 5) are not necessarily requi red to have the same flow rate. For exampl e, Flow 2 may be controlled to be greater than Flow 5 and a porti on of the priming fluid of Flow 3 may be allowed to permeate a blood purification membrane into Flow 6 with the fluid transfer means (51 and 52). Alternatively, Flow 2 may be controlled to be greater than Flow 5 and a porti on of the priming fluid of Flow 6 may be allowed to permeate a blood purification membrane into Flow 3.

In the fi rst embodi ment of the pri mi ng method, for example, the flow rate of the pri mi ng fluid can have a value of 60 for the flow rate of pri mi ng fluid to the venous side replacement fluid supply flow path (Flow 2), a value of 30 for the flow rate to the first space (Flow 3), a value of 30 for the circulation rate in the blood flow path (Flow 4), a value of 60 for the recovery rate from the arterial side replacement fluid supply flow path (Flow 5) and a value of 100 for the flow rate to the second space (Flow 6) when the overal flow rate of the priming fluid (Flows 1 and 7) are assigned a value of 100, although not limited thereto.

### <Second Embodiment of Priming Method>

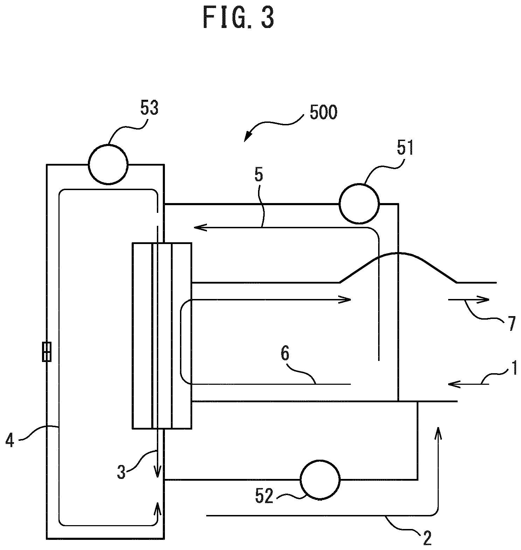

Fig. 3 is a schematic diagram showi ng a second embodi ment of the priming method of the blood purification system accordi ng to the present embodiment. The second embodi ment of the pri mi ng method includes allowing priming fluid to flow into the fluid supply flow path from the second space of the blood purifier and recovering the priming fluid from the fluid recovery flow path (Flows 1, 6 and 7), allowing a portion of the priming fluid flowing through the fluid supply flow path to flow into the arterial side replacement fluid supply flow path (Flow 5), and returni ng the priming fluid to the fluid supply flow path through the first space of the blood purifier (Flow 3) and the venous side replacement fluid supply flow path (Flow 2). As a result, the first space and the second space can be primed simultaneously. Priming fluid may be circulated (Flow 4) with the blood pump (53) by connecting the blood inlet side flow path and the blood outlet side flow path. The flow of priming fluid can be balanced at an arbitrary flow rate by independently controlling the fluid transfer means (52) of the venous side replacement fluid supply flow path, the fluid transfer means (51) of the arterial side replacement fluid supply flow path, and the blood pump (53).

In the second embodi ment of the pri mi ng method, the inflow of pri mi ng fluid to the arterial side replacement fluid supply flow path (Flow 5) and recovery of priming fluid from the venous side replacement fluid supply flow path (Flow 2) are not necessarily required to have the same flow rate. For example, Flow 5 may be controlled to be greater than Flow 2 and a porti on of the priming fluid of Flow 3 may be allowed to permeate a blood purifi cati on membrane into Flow 6 with the fluid transfer means (51 and 52). Alternatively, Flow 5 may be controlled to be greater than Flow 2 and a porti on of the priming fluid of Flow 6 may be allowed to permeate a blood purification membrane into Flow 3.

In the second embodi ment of the pri mi ng method, for example, the flow rate of the priming fluid can have a val ue of 60 for the recovery rate from the venous si de replacement fluid supply flow path (Flow 2), a value of 30 for the flow rate to the first space (Flow 3), a value of 30 for the circulation rate in the blood flow path (Flow 4), a value of 60 for the flow rate to the arterial side replacement fluid supply flow path (Flow 5), and a value of 100 for the flow rate to the second space (Flow 6) when the overall flow rate of the priming fluid (Flows 1 and 7) are assigned a value of 100, although not limited thereto.

### <Third Embodiment of Priming Method>

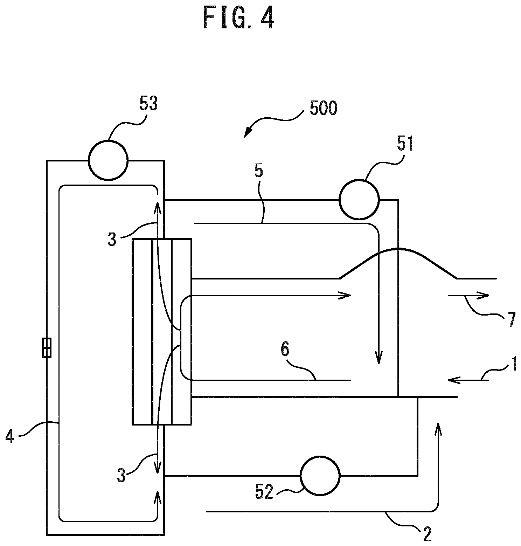

Fig. 4 is a schematic diagram showing a third embodiment of the priming method of the blood purification system accordi ng to the present embodiment. The third embodi ment of the priming method includes allowing priming fluid to flow into the fluid supply flow path from a priming fluid supply means (not shown) and recovering the priming fluid from the fluid recovery flow path through the second space of the blood purifier (Flows 1, 6 and 7), allowing a portion of the priming fluid passing through the second space to flow into first space by passing through a blood purification membrane (Flow 3), and returning the priming fluid to the fluid supply flow path through the arterial side replacement fluid supply flow path and/or the venous side replacement fluid supply flow path (Flows 2 and 5). As a result, the first space and the second space can be primed simultaneously. Priming fluid may be circulated (Flow 4) with the blood pump (53) by connecti ng the blood inlet side flow path and the blood outlet side flow path. The flow of priming fluid can be balanced at an arbitrary flow rate by independently controlling the fluid transfer means (52) of the venous side replacement fluid supply flow path, the fluid transfer means (51) of the arterial side replacement fluid supply flow path, and the blood pump (53).

In the third embodiment of the primingmethod, recovery of priming fluid from the blood circuit can be carried out through either of the arterial side replacement fluid supply flow path (Flow 5) and the venous side replacement fluid supply flow path (Flow 2). In the case of recovering from both Flow 2 and Flow 5, the flow rates thereof can be arbitrarily balanced with the fluid transfer means (51 and 52), the flow rates may be the same, Flow 5 may be made to be greater than Flow 2, or Flow 5 may be made to be greater than Flow 2.

In the third embodiment of the priming method, for example, the flow rate of the pri mi ng fluid can have a value of 60 for the recovery rate of priming fluid from the venous si de replacement fluid supply flow path (Flow 2), a value of 30 for the recovery rate from the arterial side replacement fluid supply flow path (Flow 5), a value of 190 for the flow rate of Flow 6 that flows into the second space and a value of 100 for the flow rate of Flow 6 that flows out from the second space, a value of 90 for the total flow rate from the second space to the fi rst space (Flow 3), a value of 60 for the flow rate of Flow 3 towards the fluid inlet and a value of 30 for the flow rate of Flow 3 towards the blood outlet, and a value of 30 for the circulation rate in the blood flow path (Flow 4) when the overall flow rate of the priming fluid (Flows 1 and 7) are assigned a value of 100, although not limited thereto.

### <Fourth E mbodi ment of Priming Method>

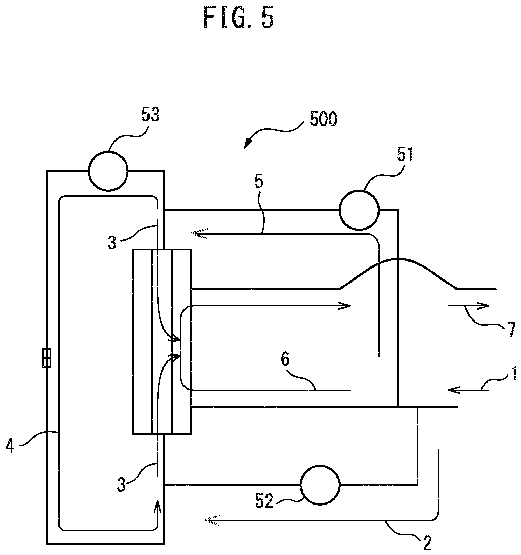

Fig. 5 is a schematic diagram showi ng a fourth embodi ment of the priming method of the blood purification system according to the present embodiment. The fourth embodiment of the pri mi ng system includes allowing pri mi ng fluid to flow into the fluid supply flow path from a pri mi ng fluid supply means (not shown) (F lows 1 and 6), allowing a porti on of the priming fluid flowing through the fluid supply flow path to flow into the first space of the blood purifier through the arteri al side replacement fluid supply flow path and/or venous side replacement fluid supply flow path (Flows 2 and 5), and allowing a portion of the priming fluid flowing through the first space to flow into the second space by passing through a blood purification membrane (Flow 3) and recovering priming fluid from the fluid supply flow path (Flow 7). As a result, the first space and the second space can be primed simultaneously. Priming fluid may be circulated (Flow 4) with the blood pump (53) by connecting the blood inlet side flow path and the blood outlet side flow path. The flow of priming fluid can be balanced at an arbitrary flow rate by independently controlling the fluid transfer means (52) of the venous side replacement fluid supply flow path, the fluid transfer means (51) of the arterial side replacement fluid supply flow path, and the blood pump (53).

In the fourth embodiment of the priming method, the supply of priming fluid to the blood circuit can be carried out through either of the arterial side replacement fluid supply flow path (Flow 5) and venous side replacement fluid supply flow path (Flow 2). In the case of supply from both Flow 2 and Flow 5, the flow rates thereof can be arbitrarily balanced with the fluid transfer means (51 and 52), the flow rates may be the same, Flow 5 may be made to be greater than Flow 2, or Flow 5 may be made to be greater than Flow 2.

In the fourth embodiment of the priming method, for example, the flow rate of the priming fluid can have a value of 30 for the flow rate of priming fluid to the venous si de replacement fluid supply flow path (Flow 2), a value of 60 for the flow rate to the arterial side replacement fluid supply flow path (Flow 5), a value of 30 for the ci rculation rate of in the blood flow path (Flow 4), a value of 90 for the total flow rate to the first space (Flow 3), a value of 30 for the flow rate of Flow 3 from the blood inlet, a value of 60 for the flow rate of Flow 3 from the blood outlet, and a value of 10 for the flow rate of Flow 6 that flows into the second space and a value of 100 for the flow rate of Flow 6 that flows out from the second space when the overall flow rate of the priming fluid (Flows 1 and 7) are assigned a value of 100, although not limited thereto.

There are no particular limitations on the priming fluid provided it is a fluid that is able to be in a state that makes it possible to initiate purification treatment, and typically replacement fluid, dialysate or physiological saline is used for blood purification treatment.

### REFERENCE SIGNS LIST

1-7 Flows
10 Blood purifier
11 First space
12 Second space
13 Blood purification membrane
21 Blood inlet side flow path
22 Blood outlet si de flow path
31 Fluid (dialysate) supply flow path
32 Fluid (dialysate) recovery flow path
41 Arterial side replacement fluid supply flow path
42 Venous side replacement fluid supply flow path
51 Liquid transfer means of arterial side replacement
fluid supply flow path
52 Liquid transfer means of venous side replacement fluid supply flow path
53 Blood pump
60 Replacement fluid pressure measuring means
70 Replacement fluid (dialysate) supply source
100 Blood purification device
200 Blood purifier
201 Arterial side end portion
202 Venous side end portion
203 Blood inlet
204 Blood outlet
207 Replacement fluid inlet
208 Main unit receptacle
209 Hollow fiber bundle
210 Sealing members
211 Dialysate inlet
212 Dialysate outlet
213 Mixing means
214 Internal space
215 Replacement fluid inlet tubular members
216 Blood purification unit
400 Blood circuit
401 Blood inlet tubular member
402 Blood outlet tubular member
403 Drip chamber
404 Tubular member for measuring pressure
405 Replacement fluid inlet tubular members
406 Priming line
407 Connecting members
408 Hydrophobic filter
500 Blood purification system

## Claims

1. An online blood purification system (500) comprising:
a blood purifier (200) that comprises a first space (11) and second space (12) partitioned by a blood purification membrane (13) and purifies blood by passing extracorporeal blood through the first space (11),
a blood inlet side flow path (21) through which the extracorporeal blood flows prior to flowing into the first space (11),
a blood outlet side flow path through (22) which the extracorporeal blood flows after having flown out from the first space (11),
a fluid supply flow path (31) for supplying a fluid to the second space (12), and
a fluid recovery flow path (32) for recovering fluid from the second space (12), **characterized in that** the online blood purification system (500) further comprises:
an arterial side replacement fluid supply flow path (41) that branches from the fluid supply flow path (31) and is connected to the blood inlet side flow path (30), and
a venous side replacement fluid supply flow path (42) that branches from the fluid supply flow path (31) and is connected to the blood outlet side flow path (22);
wherein the arterial side replacement fluid supply flow path (41) and the venous side replacement fluid supply flow path (42) respectively comprise a fluid transfer means (51 and 52) that can be independently controlled,
wherein the blood inlet side flow path (21) and the blood outlet side flow path (22) are interconnectable, and
wherein residual blood remaining in the first space (11) can be recovered by closing either the arterial side replacement fluid supply flow path (41) or the venous side replacement fluid supply flow path (42) and allowing fluid for recovering blood to flow into the bllod inlet side flow path (21) or the blood outlet side flow path (23) from the other replacement fluid supply flow path that is not closed.

2. The blood purification system (500) according to claim 1, wherein the fluid supply flow path (31) is a dialysate supply flow path for supplying dialysate to the second space (12),
the fluid recovery flow path (32) is a dialysate recovery flow path for recovering dialysate from the second space (12), and
the blood purifier (200) is a hemodialyzer capable of purifying blood by contacting the extracorporeal blood with the dialysate.

3. The blood purification system (500) according to claim 2, wherein the blood purifier (200) is a hollow fiber hemodialyzer that purifies blood by contacting dialysate flowing over the outside of hollow fibers with extracorporeal blood flowing inside the hollow fibers.

4. The blood purification system (500) according to any of claims 1 to 3, wherein the blood purification system (500) comprises a reusable blood purification device (100),
the blood purification device (100) comprises the fluid supply flow path (31), the fluid recovery flow path (32), the arterial side replacement fluid supply flow path and the venous side replacement fluid supply flow path (42),
the arterial side replacement fluid supply flow path (41)and the venous side replacement fluid supply flow path (42) supply replacement fluid directly to the extracorporeal blood, and
the blood purification device (100) further comprises a replacement fluid pressure measuring means (60) for detecting abnormalities in the flow of the extracorporeal blood.

5. The blood purification system (500) according to claim 4, wherein the replacement fluid pressure measuring means (60) directly measures the pressure of replacement fluid flowing through a region in communication with the arterial side replacement fluid supply flow path (41) and/or the venous side replacement fluid supply flow path (42).

6. The blood purification system (500) according to any of claims 1 to 5, wherein the blood purifier (200) comprises an arterial side end portion (201) having a blood inlet and a venous side end portion having a blood outlet, the blood inlet side flow path (21) is connected to the blood inlet (203) and the blood outlet side flow path (22) is connected to the blood outlet (204), and
the further comprises a replacement fluid inlet (207) in the arterial side end portion (201) and/or the venous side end portion (202), and the arterial side replacement fluid supply flow path (41) and/or the venous side replacement fluid supply flow path (42) are connected to the replacement fluid inlet (207).

7. The blood purification system (500) according to claim 6, further comprising a mixing means (213) in the arterial side end portion (201) and/or the venous side end portion (202) for uniformly mixing the extracorporeal blood and replacement fluid flowing in from the replacement fluid inlet (207).

8. The blood purification system (500) according to any of claims 1 to 7, wherein the blood purifier (200) comprises an arterial side end portion (201) comprising a blood inlet (203) and a venous side end portion (202) comprising a blood outlet (204), the blood inlet side flow path (21) is connected to the blood inlet (203), and the blood outlet side flow path (22) is connected to the blood outlet (204), and
the blood purifier (200) further comprises an internal space (214) of a prescribed volume in the arterial side end portion (201) and/or the venous side end portion (202) that is capable of capturing gas mixed in or generated during blood purification.

9. The blood purification system (500) according to any of claims 1 to 8, composed of at least one tubular member selected form the group consisting of the fluid supply flow path (31), the fluid recovery flow path (32), the arterial side replacement fluid supply flow path (41), the venous side replacement fluid supply flow path (42), the blood inlet side flow path (21) and the blood outlet side flow path (22).

10. The blood purification system (500) according to any of claims 1 to 9, wherein the fluid supply flow path (31) and the fluid recovery flow path (32) are interconnectable.

11. The blood purification system (500) according to any of claims 1 to 10, wherein the arterial side replacement fluid supply flow path (41) and the venous side replacement fluid supply flow path (42) are interconnectable.

12. The blood purification system (500) according to any of claims 1 to 11, wherein, in the fluid supply flow path (31),
the junction of the arterial side replacement fluid supply flow path (41) and the fluid supply flow path (31) is located downstream from the junction of the venous side replacement fluid supply flow path (42) and the fluid supply flow path (31).

13. The blood purification system (500) according to any of claims 1 to 12, wherein the junction of the arterial side replacement fluid supply flow path (41) and the fluid supply flow path (31) is located upstream from the junction of the venous side replacement fluid supply flow path (42) and the fluid supply flow path (31).

14. A method for priming the blood purification system (500) according to any of claims 1 to 13, comprising:
recovering priming fluid from the fluid recovery flow path (32) through the second space of the blood purifier (200), by allowing the priming fluid to flow into the fluid supply flow path (31), and
returning the priming fluid flowing through the fluid supply flow path (31) to the fluid supply flow path (31) through the first space (11) of the blood purifier (200) and the arterial side replacement fluid supply flow path (41), by allowing to flow into the venous side replacement fluid supply flow path (42).

15. A method for priming the blood purification system (500) according to any of claims 1 to 13, comprising:
recovering priming fluid from the fluid recovery flow path (32) through the second space (12) of the blood purifier (200), by allowing the priming fluid to flow into the fluid supply flow path (31), and
returning the priming fluid flowing through the fluid supply flow path (31) to the fluid supply flow path (31) through the first space (11) of the blood purifier (200) and the venous side replacement fluid supply flow path (42), by allowing to flow into the arterial side replacement fluid supply flow path (41).

16. A method for priming the blood purification system (500) according to any of claims 1 to 13, comprising:
allowing priming fluid to flow into the fluid supply flow path (31),
recovering the priming fluid from the fluid recovery flow path (32) through the second space (12) of the blood purifier (200), and returning the priming fluid passing through the second space (12) to the fluid supply flow path (31) through the arterial side replacement fluid supply flow path (41) and/or the venous side replacement fluid supply flow path (42), by allowing to pass through the blood purification membranes (13) and allowing to flow into the first space (11).

17. A method for priming the blood purification system (500) according to any of claims 1 to 13, comprising:
allowing priming fluid to flow into the fluid supply flow path (31) and allowing the priming fluid flowing through the fluid supply flow path (31) to flow into the first space (11) of the blood purifier (200) through the arterial side replacement fluid supply flow path (41) and/or the venous side replacement fluid supply flow path (42), and
recovering the priming fluid flowing through the first space (11) from the fluid recovery flow path (31) by passing through the blood purification membrane (13) and allowing to flow into the second space (12).

## Patentansprüche

1. Online-Blutreinigungssystem (500), umfassend:
einen Blutreiniger (200), der eine erste Kammer (11) und eine zweite Kammer (12) umfasst, die durch eine Blutreinigungsmembran (13) abgeteilt sind, und Blut reinigt, indem er extrakorporales Blut durch die erste Kammer (11) leitet;
eine Bluteinlassleitung (21), durch die das extrakorporale Blut fließt, bevor es in die erste Kammer (11) fließt;
eine Blutauslassleitung (22), durch die das extrakorporale Blut fließt, nachdem es aus der ersten Kammer (11) geflossen ist;
eine Flüssigkeitszufuhrleitung (31) zum Zuführen einer Flüssigkeit zur zweiten Kammer (12); und
eine Flüssigkeitsrückgewinnungsleitung (32) zur Rückgewinnung von Flüssigkeit aus der zweiten Kammer (12), **dadurch gekennzeichnet, dass** das Online-Blutreinigungssystem (500) weiterhin umfasst:
eine Substitutionsflüssigkeitszufuhrleitung der arteriellen Seite (41), die von der Flüssigkeitszufuhrleitung (31) abzweigt und an die Bluteinlassleitung (30) angeschlossen ist; und
eine Substitutionsflüssigkeitszufuhrleitung der venösen Seite (42), die von der Flüssigkeitszufuhrleitung (31) abzweigt und an die Blutauslassleitung (22) angeschlossen ist;
wobei die Substitutionsflüssigkeitszufuhrleitung der arteriellen Seite (41) und die Substitutionsflüssigkeitszufuhrleitung der venösen Seite (42) jeweils eine Flüssigkeitsübertragungseinrichtung (51 bzw. 52) umfassen,
die unabhängig gesteuert werden können;
wobei die Bluteinlassleitung (21) und die Blutauslassleitung (22) miteinander verbunden werden können; und
wobei Restblut, das in der ersten Kammer (11) zurückbleibt, zurückgewonnen werden kann, indem man entweder die Substitutionsflüssigkeitszufuhrleitung der arteriellen Seite (41) oder die Substitutionsflüssigkeitszufuhrleitung der venösen Seite (42) schließt und Flüssigkeit zur Rückgewinnung von Blut aus der anderen Substitutionsflüssigkeitszufuhrleitung, die nicht geschlossen ist, in die Bluteinlassleitung (21) oder die Blutauslassleitung (23) fließen lässt.

2. Blutreinigungssystem (500) gemäß Anspruch 1, wobei die Flüssigkeitszufuhrleitung (31) eine Dialysat-Zufuhrleitung zum Zuführen von Dialysat zur zweiten Kammer (12) ist;
die Flüssigkeitsrückgewinnungsleitung (32) eine Dialysat-Rückgewinnungsleitung zur Rückgewinnung von Dialysat aus der zweiten Kammer (12) ist; und
der Blutreiniger (200) ein Hämodialysator ist, der Blut reinigen kann, indem er das extrakorporale Blut mit dem Dialysat in Kontakt bringt.

3. Blutreinigungssystem (500) gemäß Anspruch 2, wobei der Blutreiniger (200) ein Hohlfaser-Hämodialysator ist, der Blut reinigt, indem er Dialysat, das über die Außenseite von Hohlfasern fließt, mit extrakorporalem Blut, das innerhalb der Hohlfasern fließt, in Kontakt bringt.

4. Blutreinigungssystem (500) gemäß einem der Ansprüche 1 bis 3, wobei das Blutreinigungssystem (500) eine wiederverwendbare Blutreinigungsvorrichtung (100) umfasst;
die Blutreinigungsvorrichtung (100) die Flüssigkeitszufuhrleitung (31), die Flüssigkeitsrückgewinnungsleitung (32), die Substitutionsflüssigkeitszufuhrleitung der arteriellen Seite und die Substitutionsflüssigkeitszufuhrleitung der venösen Seite (42) umfasst;
die Substitutionsflüssigkeitszufuhrleitung der arteriellen Seite (41) und die Substitutionsflüssigkeitszufuhrleitung der venösen Seite (42) Substitutionsflüssigkeit direkt zum extrakorporalen Blut zuführen; und
die Blutreinigungsvorrichtung (100) weiterhin eine Substitutionsflüssigkeitsdruckmesseinrichtung (60) zum Nachweisen von Abweichungen im Strom des extrakorporalen Blutes umfasst.

5. Blutreinigungssystem (500) gemäß Anspruch 4, wobei die Substitutionsflüssigkeitsdruckmesseinrichtung (60) direkt den Druck der Substitutionsflüssigkeit misst, die durch einen Bereich in Kommunikation mit der Substitutionsflüssigkeitszufuhrleitung der arteriellen Seite (41) und/oder der Substitutionsflüssigkeitszufuhrleitung der venösen Seite (42) fließt.

6. Blutreinigungssystem (500) gemäß einem der Ansprüche 1 bis 5, wobei der Blutreiniger (200) einen Endteil der arteriellen Seite (201) mit einem Bluteinlass und einen Endteil der venösen Seite mit einem Blutauslass umfasst, die Bluteinlassleitung (21) mit dem Bluteinlass (203) verbunden ist und die Blutauslassleitung (22) mit dem Blutauslass (204) verbunden ist; und
der weiterhin einen Substitutionsflüssigkeitseinlass (207) in dem Endteil der arteriellen Seite (201) und/oder dem Endteil der venösen Seite (202) umfasst und die Substitutionsflüssigkeitszufuhrleitung der arteriellen Seite (41) und/oder die Substitutionsflüssigkeitszufuhrleitung der venösen Seite (42) mit dem Substitutionsflüssigkeitseinlass (207) verbunden sind.

7. Blutreinigungssystem (500) gemäß Anspruch 6, weiterhin umfassend eine Mischeinrichtung (213) in dem Endteil der arteriellen Seite (201) und/oder dem Endteil der venösen Seite (202) zum gleichmäßigen Mischen des extrakorporalen Bluts und der Substitutionsflüssigkeit, die aus dem Substitutionsflüssigkeitseinlass (207) hereinfheßt.

8. Blutreinigungssystem (500) gemäß einem der Ansprüche 1 bis 7, wobei der Blutreiniger (200) einen Endteil der arteriellen Seite (201) mit einem Bluteinlass (203) und einen Endteil der venösen Seite (202) mit einem Blutauslass (204) umfasst, die Bluteinlassleitung (21) mit dem Bluteinlass (203) verbunden ist und die Blutauslassleitung (22) mit dem Blutauslass (204) verbunden ist; und
der Blutreiniger (200) weiterhin einen Innenraum (214) mit einem vorgeschriebenen Volumen in dem Endteil der arteriellen Seite (201) und/oder dem Endteil der venösen Seite (202) umfasst, der zugemischtes oder während der Blutreinigung erzeugtes Gas auffangen kann.

9. Blutreinigungssystem (500) gemäß einem der Ansprüche 1 bis 8, das aus wenigstens einem röhrenförmigen Element besteht, welches aus der Gruppe ausgewählt ist, die aus der Flüssigkeitszufuhrleitung (31), der Flüssigkeitsrückgewinnungsleitung (32), der Substitutionsflüssigkeitszufuhrleitung der arteriellen Seite (41), der Substitutionsflüssigkeitszufuhrleitung der venösen Seite (42), der Bluteinlassleitung (21) und der Blutauslassleitung (22) besteht.

10. Blutreinigungssystem (500) gemäß einem der Ansprüche 1 bis 9, wobei die Flüssigkeitszufuhrleitung (31) und die Flüssigkeitsrückgewinnungsleitung (32) miteinander verbunden werden können.

11. Blutreinigungssystem (500) gemäß einem der Ansprüche 1 bis 10, wobei die Substitutionsflüssigkeitszufuhrleitung der arteriellen Seite (41) und die Substitutionsflüssigkeitszufuhrleitung der venösen Seite (42) miteinander verbunden werden können.

12. Blutreinigungssystem (500) gemäß einem der Ansprüche 1 bis 11, wobei in der Flüssigkeitszufuhrleitung (31) sich die Verbindungsstelle der Substitutionsflüssigkeitszufuhrleitung der arteriellen Seite (41) und der Flüssigkeitszufuhrleitung (31) stromabwärts von der Verbindungsstelle der Substitutionsflüssigkeitszufuhrleitung der venösen Seite (42) und der Flüssigkeitszufuhrleitung (31) befindet.

13. Blutreinigungssystem (500) gemäß einem der Ansprüche 1 bis 12, wobei sich die Verbindungsstelle der Substitutionsflüssigkeitszufuhrleitung der arteriellen Seite (41) und der Flüssigkeitszufuhrleitung (31) stromaufwärts von der Verbindungsstelle der Substitutionsflüssigkeitszufuhrleitung der venösen Seite (42) und der Flüssigkeitszufuhrleitung (31) befindet.

14. Verfahren zur Vorbereitung des Blutreinigungssystems (500) gemäß einem der Ansprüche 1 bis 13, umfassend:
Rückgewinnung von Vorbereitungsflüssigkeit aus der Flüssigkeitsrückgewinnungsleitung (32) über die zweite Kammer des Blutreinigers (200), indem man die Vorbereitungsflüssigkeit in die Flüssigkeitszufuhrleitung (31) fließen lässt; und
Rückführung der durch die Flüssigkeitszufuhrleitung (31) fließenden Vorbereitungsflüssigkeit zu der Flüssigkeitszufuhrleitung (31) über die erste Kammer (11) des Blutreinigers (200) und die Substitutionsflüssigkeitszufuhrleitung der arteriellen Seite (41), indem man sie in die Substitutionsflüssigkeitszufuhrleitung der venösen Seite (42) fließen lässt.

15. Verfahren zur Vorbereitung des Blutreinigungssystems (500) gemäß einem der Ansprüche 1 bis 13, umfassend:
Rückgewinnung von Vorbereitungsflüssigkeit aus der Flüssigkeitsrückgewinnungsleitung (32) über die zweite Kammer (12) des Blutreinigers (200), indem man die Vorbereitungsflüssigkeit in die Flüssigkeitszufuhrleitung (31) fließen lässt; und
Rückführung der durch die Flüssigkeitszufuhrleitung (31) fließenden Vorbereitungsflüssigkeit zu der Flüssigkeitszufuhrleitung (31) über die erste Kammer (11) des Blutreinigers (200) und die Substitutionsflüssigkeitszufuhrleitung der venösen Seite (42), indem man sie in die Substitutionsflüssigkeitszufuhrleitung der arteriellen Seite (41) fließen lässt.

16. Verfahren zur Vorbereitung des Blutreinigungssystems (500) gemäß einem der Ansprüche 1 bis 13, umfassend:
Fließenlassen von Vorbereitungsflüssigkeit in die Flüssigkeitszufuhrleitung (31);
Rückgewinnung der Vorbereitungsflüssigkeit aus der Flüssigkeitsrückgewinnungsleitung (32) über die zweite Kammer (12) des Blutreinigers (200); und
Rückführung der durch die zweite Kammer (12) fließenden Vorbereitungsflüssigkeit zu der Flüssigkeitszufuhrleitung (31) über die Substitutionsflüssigkeitszufuhrleitung der arteriellen Seite (41) und/oder die Substitutionsflüssigkeitszufuhrleitung der venösen Seite (42), indem man sie durch die Blutreinigungsmembranen (13) treten und in die erste Kammer (11) fließen lässt.

17. Verfahren zur Vorbereitung des Blutreinigungssystems (500) gemäß einem der Ansprüche 1 bis 13, umfassend:
Fließenlassen von Vorbereitungsflüssigkeit in die Flüssigkeitszufuhrleitung (31) Fließenlassen der durch die Flüssigkeitszufuhrleitung (31) fließenden Vorbereitungsflüssigkeit in die erste Kammer (11) des Blutreinigers (200) über die Substitutionsflüssigkeitszufuhrleitung der arteriellen Seite (41) und/oder die Substitutionsflüssigkeitszufuhrleitung der venösen Seite (42); und
Rückgewinnung der durch die erste Kammer (11) fließenden Vorbereitungsflüssigkeit aus der Flüssigkeitszufuhrleitung (31), indem man sie durch die Blutreinigungsmembran (13) treten und in die zweite Kammer (12) fließen lässt.

## Revendications

1. Système de purification de sang en ligne (500) comprenant :
un purificateur de sang (200) qui comprend un premier espace (11) et un deuxième espace (12) séparés par une membrane de purification de sang (13) et qui purifie le sang en faisant passer le sang extracorporel à travers le premier espace (11),
un trajet d'écoulement côté entrée de sang (21) à travers lequel le sang extracorporel s'écoule avant de s'écouler dans le premier espace (11),
un trajet d'écoulement côté sortie de sang (22) à travers lequel le sang extracorporel s'écoule après avoir circulé hors du premier espace (11),
un trajet d'écoulement d'alimentation en fluide (31) pour fournir un fluide au deuxième espace (12), et
un trajet d'écoulement de récupération de fluide (32) pour récupérer le fluide du deuxième espace (12), **caractérisé en ce que** le système de purification de sang en ligne (500) comprend en outre :
un trajet d'écoulement d'alimentation en fluide de remplacement côté artériel (41) qui bifurque du trajet d'écoulement d'alimentation en fluide (31) et qui est relié au trajet d'écoulement côté entrée de sang (30), et
un trajet d'écoulement d'alimentation en fluide de remplacement côté veineux (42) qui bifurque du trajet d'écoulement d'alimentation en fluide (31) et qui est relié au trajet d'écoulement côté sortie de sang (22) ;
dans lequel le trajet d'écoulement d'alimentation en fluide de remplacement côté artériel (41) et le trajet d'écoulement d'alimentation en fluide de remplacement côté veineux (42) comprennent respectivement des moyens de transfert de fluide (51 et 52) qui peuvent être commandés indépendamment,
dans lequel le trajet d'écoulement côté entrée de sang (21) et le trajet d'écoulement côté sortie de sang (22) pouvant être reliés entre eux, et
dans lequel le sang résiduel restant dans le premier espace (11) peut être récupéré en fermant soit le trajet d'écoulement d'alimentation en fluide de remplacement côté artériel (41) soit le trajet d'écoulement d'alimentation en fluide de remplacement côté veineux (42) et en permettant au fluide de récupération de sang de s'écouler dans le trajet d'écoulement côté entrée de sang (21) ou le trajet d'écoulement côté sortie de sang (23) à partir de l'autre trajet d'écoulement d'alimentation en fluide de remplacement qui n'est pas fermé.

2. Système de purification de sang (500) selon la revendication 1, dans lequel le trajet d'écoulement d'alimentation en fluide (31) est un trajet d'écoulement d'alimentation en dialysat pour fournir le dialysat au deuxième espace (12),
le trajet d'écoulement de récupération de fluide (32) est un trajet d'écoulement de récupération de dialysat pour récupérer le dialysat du deuxième espace (12), et
le purificateur de sang (200) est un hémodialyseur capable de purifier le sang en mettant en contact le sang extracorporel avec le dialysat.

3. Système de purification de sang (500) selon la revendication 2, dans lequel le purificateur de sang (200) est un hémodialyseur à fibres creuses qui purifie le sang en mettant en contact le dialysat s'écoulant sur l'extérieur des fibres creuses avec le sang extracorporel s'écoulant à l'intérieur des fibres creuses.

4. Système de purification de sang (500) selon l'une des revendications 1 à 3, dans lequel le système de purification de sang (500) comprend un dispositif de purification de sang réutilisable (100),
le dispositif de purification de sang (100) comprend le trajet d'écoulement d'alimentation en fluide (31), le trajet d'écoulement de récupération de fluide (32), le trajet d'écoulement d'alimentation en fluide de remplacement côté artériel et le trajet d'écoulement d'alimentation en fluide de remplacement côté veineux (42),
le trajet d'écoulement d'alimentation en fluide de remplacement côté artériel (41) et le trajet d'écoulement d'alimentation en fluide de remplacement côté veineux (42) fournissent le fluide de remplacement directement au sang extracorporel, et
le dispositif de purification de sang (100) comprend en outre un moyen de mesure de pression de fluide de remplacement (60) pour détecter des anomalies dans l'écoulement du sang extracorporel.

5. Système de purification de sang (500) selon la revendication 4, dans lequel le moyen de mesure de pression de fluide de remplacement (60) mesure directement la pression du fluide de remplacement s'écoulant à travers une région en communication avec le trajet d'écoulement d'alimentation en fluide de remplacement côté artériel (41) et/ou le trajet d'écoulement d'alimentation en fluide de remplacement côté veineux (42).

6. Système de purification de sang (500) selon l'une des revendications 1 à 5, dans lequel le purificateur de sang (200) comprend une partie d'extrémité côté artériel (201) ayant une entrée de sang et une partie d'extrémité côté veineux ayant une sortie de sang, le trajet d'écoulement côté entrée de sang (21) est relié à l'entrée de sang (203) et le trajet d'écoulement côté sortie de sang (22) est relié à la sortie de sang (204), et
comprend en outre une entrée de fluide de remplacement (207) dans la partie d'extrémité côté artériel (201) et/ou dans la partie d'extrémité côté veineux (202), et le trajet d'écoulement d'alimentation en fluide de remplacement côté artériel (41) et/ou le trajet d'écoulement d'alimentation en fluide de remplacement côté veineux (42) sont reliés à l'entrée de fluide de remplacement (207).

7. Système de purification de sang (500) selon la revendication 6, comprenant en outre un moyen de mélange (213) dans la partie d'extrémité côté artériel (201) et/ou la partie d'extrémité côté veineux (202) pour mélanger uniformément le sang extracorporel et le fluide de remplacement s'écoulant à partir de l'entrée de fluide de remplacement (207).

8. Système de purification de sang (500) selon l'une des revendications 1 à 7, dans lequel le purificateur de sang (200) comprend une partie d'extrémité côté artériel (201) comprenant une entrée de sang (203) et une partie d'extrémité côté veineux (202) comprenant une sortie de sang (204), le trajet d'écoulement côté entrée de sang (21) est relié à l'entrée de sang (203), et le trajet d'écoulement côté sortie de sang (22) est relié à la sortie de sang (204), et
le purificateur de sang (200) comprend en outre un espace interne (214) d'un volume prescrit dans la partie d'extrémité côté artériel (201) et/ou la partie d'extrémité côté veineux (202) qui est capable de capturer le gaz mélangé ou généré lors de la purification de sang.

9. Système de purification du sang (500) selon l'une des revendications 1 à 8, composé d'au moins un élément tubulaire sélectionné dans le groupe constitué du trajet d'écoulement d'alimentation en fluide (31), du trajet d'écoulement de récupération de fluide (32), du trajet d'écoulement d'alimentation en fluide de remplacement côté artériel (41), du trajet d'écoulement d'alimentation en fluide de remplacement côté veineux (42), du trajet d'écoulement côté entrée de sang (21) et du trajet d'écoulement côté sortie de sang (22).

10. Système de purification de sang (500) selon l'une des revendications 1 à 9, dans lequel le trajet d'écoulement d'alimentation en fluide (31) et le trajet d'écoulement de récupération de fluide (32) peuvent être reliés entre eux.

11. Système de purification de sang (500) selon l'une des revendications 1 à 10, dans lequel le trajet d'écoulement d'alimentation en fluide de remplacement côté artériel (41) et le trajet d'écoulement d'alimentation en fluide de remplacement côté veineux (42) peuvent être reliés entre eux.

12. Système de purification de sang (500) selon l'une des revendications 1 à 11, dans lequel, dans le trajet d'écoulement d'alimentation en fluide (31), la jonction du trajet d'écoulement d'alimentation en fluide de remplacement côté artériel (41) et du trajet d'écoulement d'alimentation en fluide (31) est située en aval de la jonction du trajet d'écoulement d'alimentation en fluide de remplacement côté veineux (42) et du trajet d'écoulement d'alimentation en fluide (31).

13. Système de purification de sang (500) selon l'une des revendications 1 à 12, dans lequel la jonction du trajet d'écoulement d'alimentation en fluide de remplacement côté artériel (41) et du trajet d'écoulement d'alimentation en fluide (31) est située en amont de la jonction du trajet d'écoulement d'alimentation en fluide de remplacement côté veineux (42) et du trajet d'écoulement d'alimentation en fluide (31).

14. Procédé d'amorçage du système de purification de sang (500) selon l'une des revendications 1 à 13, comprenant le fait :
de récupérer le fluide d'amorçage du trajet d'écoulement de récupération de fluide (32) à travers le deuxième espace du purificateur de sang (200), en permettant au fluide d'amorçage de s'écouler dans le trajet d'écoulement d'alimentation en fluide (31), et
de renvoyer le fluide d'amorçage s'écoulant à travers le trajet d'écoulement d'alimentation en fluide (31) au trajet d'écoulement d'alimentation en fluide (31) à travers le premier espace (11) du purificateur de sang (200) et le trajet d'écoulement d'alimentation en fluide de remplacement côté artériel (41), en lui permettant de s'écouler dans le trajet d'écoulement d'alimentation en fluide de remplacement côté veineux (42).

15. Procédé d'amorçage du système de purification de sang (500) selon l'une des revendications 1 à 13, comprenant le fait :
de récupérer le fluide d'amorçage du trajet d'écoulement de récupération de fluide (32) à travers le deuxième espace (12) du purificateur de sang (200), en permettant au fluide d'amorçage de s'écouler dans le trajet d'écoulement d'alimentation en fluide (31), et
de renvoyer le fluide d'amorçage s'écoulant à travers le trajet d'écoulement d'alimentation en fluide (31) au trajet d'écoulement d'alimentation en fluide (31) à travers le premier espace (11) du purificateur de sang (200) et le trajet d'écoulement d'alimentation en fluide de remplacement côté veineux (42), en lui permettant de s'écouler dans le trajet d'écoulement d'alimentation en fluide de remplacement côté artériel (41).

16. Procédé d'amorçage du système de purification de sang (500) selon l'une des revendications 1 à 13, comprenant le fait :
de permettre au fluide d'amorçage de s'écouler dans le trajet d'écoulement d'alimentation en fluide (31),
de récupérer le fluide d'amorçage du trajet d'écoulement de récupération de fluide (32) à travers le deuxième espace (12) du purificateur de sang (200), et
de renvoyer le fluide d'amorçage passant à travers le deuxième espace (12) au trajet d'écoulement d'alimentation en fluide (31) à travers le trajet d'écoulement d'alimentation en fluide de remplacement côté artériel (41) et/ou le trajet d'écoulement d'alimentation en fluide de remplacement côté veineux (42), en lui laissant passer à travers les membranes de purification de sang (13) et en lui permettant de s'écouler dans le premier espace (11).

17. Procédé d'amorçage du système de purification de sang (500) selon l'une des revendications 1 à 13, comprenant le fait :
de permettre au fluide d'amorçage de s'écouler dans le trajet d'écoulement d'alimentation en fluide (31) et de permettre au fluide d'amorçage de s'écouler à travers le trajet d'écoulement d'alimentation en fluide (31) pour s'écouler dans le premier espace (11) du purificateur de sang (200) à travers le trajet d'écoulement d'alimentation en fluide de remplacement côté artériel (41) et/ou le trajet d'écoulement d'alimentation en fluide de remplacement côté veineux (42), et
de récupérer le fluide d'amorçage s'écoulant à travers le premier espace (11) du trajet d'écoulement de récupération de fluide (31) en passant à travers la membrane de purification de sang (13) et en lui permettant de s'écouler dans le deuxième espace (12).
